(19) Europäisches Patentamt
European Patent Office
Office européen des brevets

(11) Publication number: **0 411 720 B1**

(12) # EUROPEAN PATENT SPECIFICATION

(45) Date of publication of patent specification: **18.01.95**

(51) Int. Cl.6: **C07C 323/48**, C07C 317/14, C07C 291/08, A01N 33/26

(21) Application number: **90202126.0**

(22) Date of filing: **03.08.90**

The file contains technical information submitted after the application was filed and not included in this specification

(54) **Biocidal compounds.**

(30) Priority: **04.08.89 GB 8917848**

(43) Date of publication of application:
**06.02.91 Bulletin 91/06**

(45) Publication of the grant of the patent:
**18.01.95 Bulletin 95/03**

(84) Designated Contracting States:
**CH DE FR GB IT LI NL**

(56) References cited:

**RECUEIL, JOURNAL OF THE ROYAL NETHERLANDS CHEMICAL SOCIETY, vol. 90, no. 7,1971, DEN HAAG, NL, pages 641 - 653; F. HAVERKATE ET AL.: "INTERACTION OF BENZENEDIAZOCYANIDES WITH FUNGAL SPORES AND ITS RELATION TO THEIR STABILITY IN LIGHT ."**

**RECUEIL DES TRAVAUX CHIMIOUES DES PAYS-BAS. vol. 87, no. 9, September 1968, DEN HAAG, NL, pages 833-956; C. W. PLUIJGERS ET AL.: "OXIDATION PRODUCTS OF 1-PHENYLTHIOSEMICARBAZIDES AND THEIR ANTIFUNGAL ACTIVITY"**

(73) Proprietor: **SHELL INTERNATIONALE RESEARCH MAATSCHAPPIJ B.V.
Carel van Bylandtlaan 30
NL-2596 HR Den Haag (NL)**

(72) Inventor: **Gray, Andrew Clement Gripper
63 New Road
London E1 1HH (GB)**
Inventor: **Naisby, Thomas Webster
79 Gadby Road
Sittingbourne,
Kent (GB)**
Inventor: **Turner, Susan Jayne
6 Peregrine Drive
Sittingbourne,
Kent ME10 4TZ (GB)**
Inventor: **Machin, Tarnia Michelle
25 Farm Crescent
Sittingbourne,
Kent ME10 4OD (GB)**

(74) Representative: **Allam, Peter Clerk et al
LLOYD WISE, TREGEAR & CO.
Norman House
105-109 Strand
London WC2R 0AE (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

EP 0 411 720 B1

**JOURNAL OF THE CHEMICAL SOCIETY, CHEMICAL COMMUNICATIONS, no. 5, 1984, LETCHWORTH, GB, pages 323 - 324; R. FRUTTERO ET AL.: "A DIRECTED SYNTHESIS OF ALKYL, ARYL, AND HETEROARYL-ONN-AZOXYCYANIDES"**

## Description

This invention relates to novel compounds having an azo substituent, to their use as fungicidal and/or bactericidal and/or nematicidal agents, to fungicidal and/or bactericidal and/or nematicidal compositions containing such compounds, and to the preparation of such compounds.

Calvatic acid, (4-(cyano-N,N,O-azoxy)benzoic acid) and a limited number of analogous compounds, have been investigated in respect of their antibacterial and antifungal properties. Thus, in Trans. Mycol. Soc. Japan, 23, p. 225-234, 1982, calvatic acid and its methyl ester are described as having antibacterial and antifungal activity. In Eur. J. Med. Chem - Chimica Therapeutica, Jan-Feb. 1977 - 12 No. 1, p. 59-62, the preparation and screening of further analogues is described. The compounds made and tested were of the following formula :-

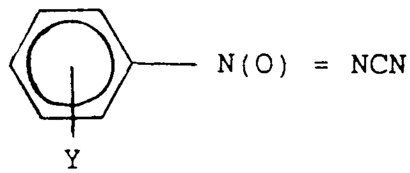

Y : 2-Cl; 3-Cl; 4-Cl;
2-Br; 3-Br; 4-Br;
2-$NO_2$; 3-$NO_2$; 4-$NO_2$;
2-$OCH_3$; 3-$OCH_3$; 4-$OCH_3$;
H;
p-$N(CH_3)_2$

Recueil, the Journal of the Royal Netherlands Chemical Society,vol. 90, no. 7, p.641-653 (1971), discloses 4-methylsulphonyl benzenediazocyanide, the preparation of that and other substituted benzene diazocyanides by diazotisation of corresponding anilines followed by treatment with aqueous KCN, their action against Fusarium culmorum in the dark, and the rate of photodecomposition of the test compounds.

Antibacterial and antifungal properties of 4-carboxyphenylazoxycyanide-dimethylsulphoxide are described in Acta Crystallogr., Sect. B, 1975, B31(8) p.2151-3.

The antibacterial properties of certain further compounds are described in Japanese Patent application (Kokai) J5 2071444 (Takara Shuzo KK). The compounds are said (in Chemical Abstract No. 87:167770) to be compounds of the formula given above, where Y is 2-$CH_3$; 3-$CH_3$; 3-COOH; 3-Cl; 3,4-$Cl_2$; and 2,5-$CH_3$,Cl).

In the Journal of Antibiotics 6/1986, p.864-8, the preparation and bactericidal and fungicidal screening of 2-(cyano-N,N,O-azoxy)benzoic acid is described but it is said to show no relevant activity against the tested fungi, and to show low activity against bacteria.

In U.S. Patent Nos. 4558040 and 4550121 there is described the miticidal activity of (2-alkyl-3,4-dihydro-2H-1-benzoypyran-8-yl)diazenecarboxylic acid esters and (2-substituted-2,3-dihydrobenzo-furan-7-yl)diazenecarboxylicacid esters.

The present invention is based upon the discovery of certain novel compounds, and the subsequent discovery of their effectiveness in combating fungi, including plant pathogenic fungi, bacteria and yeasts, and nematodes.

According to a first aspect of the present invention there is provided a method of combating a bacterium and/or nematode at a locus, which method comprises treating a locus with a compound of the general formula

R-N = NX (I)

or an N-oxide thereof, where R represents a substituted phenyl group, at least one substituent thereof being a group of general formula -$S(O)_m$-Z where m represents 0, 1 or 2 and Z represents a hydrogen atom or an optionally substituted alkyl, cycloalkyl, aryl, aralkyl or amino group; and X represents a cyano group, a group -COOH or a salt, ester or amido derivative thereof.

Unless otherwise specified in this specification, an alkyl group, including the alkyl linkage of an aralkyl group, may be linear or branched and suitably contains up to 10, preferably up to 6, and most preferably up to 4, carbon atoms, a preferred example being methyl. A cycloalkyl group preferably has 3 to 6 carbon atoms, a preferred example being cyclopropyl. An aryl group, including the aryl part of an aralkyl group, is

preferably phenyl.

Unless otherwise stated in this specification, when any groups are designated as being optionally substituted, the substituent groups which are optionally present may be any of those customarily employed in the development of biocidal compounds, and/or the modification of such compounds to influence their structure/activity, persistence, penetration or other property. In relation to an alkyl or cycloalkyl group, specific examples of such substituents may include halogen, especially fluorine, chlorine or bromine atoms, and phenyl, alkoxy, hydroxy, cyano and (alkyl)amino groups. In relation to an aryl moiety, optional substituents may include halogen atoms, for example fluorine, chlorine, bromine and iodine atoms, and nitro, cyano, alkoxy, hydroxy, (alkyl)amino, alkyl, and haloalkyl (especially $CF_3$) groups.

More specifically, Z preferably represents an optionally substituted alkyl, cycloalkyl, aralkyl or amino group. More preferably, Z represents a $C_{1-4}$ alkyl group, a $C_{3-6}$ cycloalkyl group, a benzyl group or a group of general formula $NR^1R^2$ where each of $R^1$ and $R^2$ independently represents a hydrogen atom, or an optionally substituted phenyl or alkyl group, or together may represent an alkylene chain which is optionally interrupted by an oxygen atom. Preferably, $R^1$ represents a hydrogen atom and $R^2$ represents a hydrogen atom, or a phenyl or halophenyl group, or an alkyl group optionally substituted by a group of general formula $-CO_2R^3$ where $R^3$ represents a hydrogen atom or an alkyl group; or $R^1$ and $R^2$ together represent a group of formula $-(CH_2)_2-O-(CH_2)_2-$ (the group Z thus being an N-morpholino group).

Preferably, the phenyl group R is substituted by one such group $-S(O)_m-Z$, and is optionally substituted by one or more further substituents for example halogen atom(s). Preferably, R represents a group of general formula

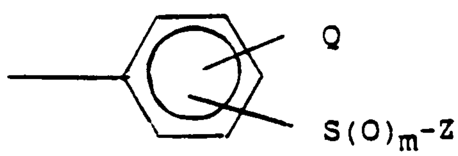

where Q represents a halogen, especially chlorine, atom, or most preferably, a hydrogen atom.

Preferably, m represents 0 or 2.

Preferably, the compounds of the general formula I are N-oxidised.

Preferably, X represents a cyano group, a group COOH or a group $-COOZ^1$ where $Z^1$ represents a $C_{1-4}$ alkyl, alkenyl or alkynyl group, for example methyl, ethyl, allyl or propargyl. Most preferably, X represents a cyano group.

It should be noted that compounds of the general formula I may exist as cis or trans isomers, and that the scope of the present invention in all its aspects covers such isomers, isolated or together.

It should also be noted that N-oxidised compounds of general formula could be in any of the following isoelectronic forms:

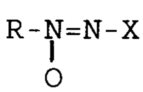 (IA) 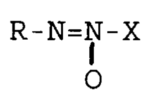 (IB) 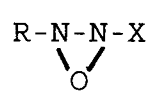 (IC)

It is believed, on the basis of NMR analysis, that form IA is the preferred/dominant form, rather than form IB or IC, but it should be noted that the scope of the present invention covers any such forms.

In accordance with a second aspect of the present invention there is provided a method of combating a fungus and/or yeast at a locus, which comprises treating the locus with a compound of general formula I, or N-oxide thereof, as defined above with the proviso that, in an un-N-oxidised compound of formula I, when m is 2, Z is other than a methyl group.

In the methods of the invention as described above, the locus may be an agricultural or horticultural locus, for example plants subject to attack, seeds of such plants or the medium in which such plants are growing or are to be grown, or a non-living organic locus such as crude oil, an oil-derived liquid fuel, or lubricant, or a paint, detergent or textile. In relation to an agricultural or horticultural locus, compounds of the present invention have been shown to exhibit activity against a range of important fungi, including vine downy mildew, vine grey mould, wheat leafspot, barley powdery mildew, tomato early blight, wheat eyespot, seedling wheat blight and wheat brown rust, and against the rice root nematode Meloidogyne graminicola.

Particularly interesting activity has been found against vine downy mildew. A locus as described above may suitably be treated with a compound I at an application rate in the range 0.05-4 Kg/ha, preferably 0.1-1 Kg/ha. In relation to a non-agrochemical locus, compounds of the present invention have been shown to exhibit activity against certain filamentous fungi, gram-positive and gram-negative bacteria (including the sulphate-reducing bacterium Desulfovibrio sp.) and the yeast Saccaromyces cerevisiae.

The invention further provides the use, as a bactericide and/or nematicide, of a compound of the general formula I, or N-oxide thereof, or the use as a biocide of a compound of formula I, or N-oxide thereof, wherein, in un-N-oxidised form, when m is 2, Z is other than methyl.

Further in accordance with the invention there is provided a biocidal composition which comprises a carrier and, as active ingredient, a compound of general formula I or N-oxide thereof, wherein, in un-N-oxidised form, when m is 2, Z is other than methyl.

A carrier in a composition according to the invention is any material with which the active ingredient is formulated to facilitate application to the locus to be treated, or to facilitate storage, transport or handling. A carrier may be a solid or a liquid, including a material which is normally gaseous but which has been compressed to form a liquid, and any of the carriers normally used in formulating biocidal compositions may be used. Preferably compositions according to the invention contain 0.5 to 95% by weight of active ingredient.

Suitable solid carriers include natural and synthetic clays and silicates, for example natural silicas such as diatomaceous earths; magnesium silicates, for example talcs; magnesium aluminium silicates, for example attapulgites and vermiculites; aluminium silicates, for example kaolinites, montmorillonites and micas; calcium carbonate; calcium sulphate; ammonium sulphate; synthetic hydrated silicon oxides and synthetic calcium or aluminium silicates; elements, for example carbon and sulphur; natural and synthetic resins, for example coumarone resins, polyvinyl chloride, and styrene polymers and copolymers; solid polychlorophenols; bitumen; waxes; and solid fertilisers, for example superphosphates.

Suitable liquid carriers include water; alcohols, for example isopropanol and glycols; ketones, for example acetone, methyl ethyl ketone, methyl isobutyl ketone and cyclohexanone; ethers; aromatic or araliphatic hydrocarbons, for example benzene, toluene and xylene; petroleum fractions, for example kerosene and light mineral oils; chlorinated hydrocarbons, for example carbon tetrachloride, perchloroethylene and trichloroethane. Mixtures of different liquids are often suitable.

Compositions are often formulated and transported in a concentrated form which is subsequently diluted by the user before application. The presence of small amounts of a carrier which is a surface-active agent facilitates this process of dilution. Thus preferably at least one carrier in a composition according to the invention is a surface-active agent. For example the composition may contain at least two carriers, at least one of which is a surface-active agent.

A surface-active agent may be an emulsifying agent, a dispersing agent or a wetting agent; it may be nonionic or ionic. Examples of suitable surface-active agents include the sodium or calcium salts of polyacrylic acids and lignin sulphonic acids; the condensation of fatty acids or aliphatic amines or amides containing at least 12 carbon atoms in the molecule with ethylene oxide and/or propylene oxide; fatty acid esters of glycerol, sorbitol, sucrose or pentaerythritol; condensates of these with ethylene oxide and/or propylene oxide; condensation products of fatty alcohol or alkyl phenols, for example p-octylphenol or p-octylcresol, with ethylene oxide and/or propylene oxide; sulphates or sulphonates of these condensation products; alkali or alkaline earth metal salts, preferably sodium salts, of sulphuric or sulphonic acid esters containing at least 10 carbon atoms in the molecule, for example sodium lauryl sulphate, sodium secondary alkyl sulphates, sodium salts of sulphonated castor oil, and sodium alkylaryl sulphonates such as dodecylbenzene sulphonate; and polymers of ethylene oxide and copolymers of ethylene oxide and propylene oxide.

The compositions of the invention may for example be formulated as wettable powders, dusts, granules, solutions, emulsifiable concentrates, emulsions, suspension concentrates and aerosols. Wettable powders usually contain 25, 50 or 75% w of active ingredient and usually contain in addition to solid inert carrier, 3-10% w of a dispersing agent and, where necessary, 0-10% w of stabiliser(s) and/or other additives such as penetrants or stickers. Dusts are usually formulated as a dust concentrate having a similar composition to that of a wettable powder but without a dispersant, and are diluted in the field with further solid carrier to give a composition usually containing 0.5-10% w of active ingredient. Granules are usually prepared to have a size between 10 and 100 BS mesh (1.676 - 0.152 mm), and may be manufactured by agglomeration or impregnation techniques. Generally, granules will contain 0.5-75% w active ingredient and 0-10% w of additives such as stabilisers, surfactants, slow release modifiers and binding agents. The so-called "dry flowable powders" consist of relatively small granules having a relatively high concentration of active ingredient. Emulsifiable concentrates usually contain, in addition to a solvent and, when necessary, co-solvent, 10-50% w/v active ingredient, 2-20% w/v emulsifiers and 0-20% w/v of other additives such as stabilisers, penetrants and corrosion inhibitors. Suspension concentrates are usually compounded so as to obtain a stable, non-sedimenting flowable product and usually contain 10-75% w active ingredient, 0.5-15% w of dispersing agents, 0.1-10% w of suspending agents such as protective colloids and thixotropic agents, 0-10% w of other additives such as defoamers, corrosion inhibitors, stabilisers, penetrants and stickers, and water or an organic liquid in which the active ingredient is substantially insoluble; certain organic solids or inorganic salts may be present dissolved in the formulation to assist in preventing sedimentation or as anti-freeze agents for water.

Aqueous dispersions and emulsions, for example compositions obtained by diluting a wettable powder or a concentrate according to the invention with water, also lie within the scope of the invention. The said emulsions may be of the water-in-oil or of the oil-in-water type, and may have a thick "mayonnaise" - like consistency.

The composition of the invention may also contain other ingredients, for example other compounds possessing herbicidal, insecticidal or fungicidal properties.

The present invention also concerns novel compounds of general formula I, or N-oxides thereof wherein, in un-N-oxidised forms, when m is 2, Z is other than a methyl group.

In accordance with a further aspect of the invention there is provided a process for the preparation of a compound of general formula I, or an N-oxide thereof, which comprises reacting a compound of the general formula

R-N=O (II)

with cyanamide, to form an N-oxide compound of formula I wherein X represents a cyano group; and optionally derivatising that compound to produce a further compound of formula I.

Suitably, the reaction takes place in the presence of an organic solvent, preferably a halogenated hydrocarbon, for example chloroform, and in the presence of iodobenzene diacetate. The reaction is preferably effected at a temperature in the range - 20 °C to 50 °C.

Derivatisation of the compound of formula I may, for example, be effected by standard hydrolysis, in the presence of a strong acid or a strong base, to convert the cyano group to a carboxy group, or, stopping the reaction at an intermediate stage, an amido group.

Esters may be prepared by standard esterification of the resultant carboxylic acid or by acid alcoholysis of the cyano compound to form the acid salt of the imidate ester, which is reacted with water, suitably at ambient temperature, to yield the ester. Alternatively, esters may be prepared by the following general route, described in greater detail in U.S. Patent Nos. 4558040 and 4550121, (R representing the aryl group):

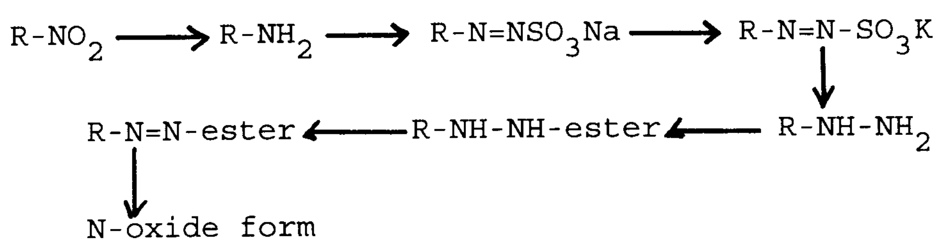

The latter two compounds may be converted to other compounds of formula I, for example amides, acids and nitriles, by standard methods.

A compound of formula II may be prepared as follows:

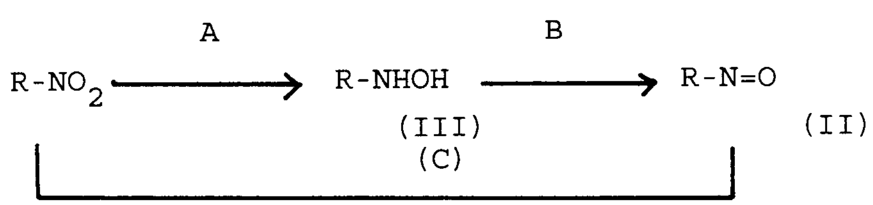

Reaction A may, for example, be effected by reaction of the nitro compound with hydrazine hydrate, in the presence of a hydrogen transfer catalyst, for example rhodium on carbon, suitably in the presence of an inert polar organic solvent, for example tetrahydrofuran, preferably at a temperature in the range -20°C to 50°C conveniently at ambient temperature, or be effected using water, stannous chloride as reducing agent, an inert, polar organic solvent, for example tetrahydrofuran, under an inert atmosphere, for example nitrogen, in the presence of sodium acetate, suitably at ambient temperature.

Reaction B may suitably be effected by treatment of the hydroxylamine derivative with an oxidising agent, for example an $Fe^{3+}$ compound, suitably ferric chloride. The reaction may be effected in a mixed water/polar organic solvent, for example methanol, preferably with cooling.

Reaction C may be effected by irradiating the nitro compound, which is preferably dissolved in an inert organic solvent, for example benzene. The irradiation may be effected using a medium pressure mercury lamp.

The nitro, hydroxylamine and nitroso compounds are known or else may be prepared from known compounds by standard methods. Any such compounds which are novel constitute further aspects of the invention, together with methods for their preparation.

Novel unoxidised compounds of formula I may be made by analogous method to that described in U.S. Patent No. 2910463 and Med. Chem- Chim.Ther., 1982-17, No. 5,p. 482-4, wherein diazotisation of an amine compound is carried out, and the resulting diazotised compound is cyanated, in the following manner.

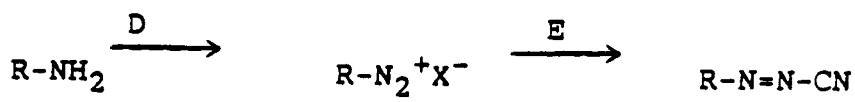


(IV) (I)

where $X^-$ is an anion derived from a mineral acid. Optionally, a resultant compound I may be derivatised. for example oxidised under standard conditions to yield an N-oxide compound of general formula I and/or to derive a sulphone, from a sulphoxide or un-S-oxidised compound of general formula I. S-oxidation of a compound of formula I prepared by the "cyanamide" route described above may, of course, also be effected thus.

A typical oxidation method uses a "peroxy" compound, for example hydrogen peroxide, or a peroxycarboxylic acid in a suitable inert organic solvent, for example a halogenated hydrocarbon, such as dichloromethane, at a temperature in the range -20 to 60°C. Suitable "peroxy" acids include peroxytrifluoroacetic acid and metachloroperbenzoic acid.

For reaction D, standard diazotisation conditions are employed, for example. a low temperature, suitably - 10°C to 20°C, and sodium nitrite in an aqueous mineral acid.

For reaction E, cyanation is suitably effected by treating the compound of general formula IV with an alkali metal cyanide, for example sodium cyanide, suitably at a low temperature, for example -20 to +20°C, removing the aqueous layer, adding a halogenated hydrocarbon, for example carbon tetrachloride, and heating the organic layer, suitably at a temperature in the range 40-100°C, preferably under reflux.

Other methods suitable for preparing compounds, for formula I, and further descriptions of the methods described herein, may be found in The Journal of Antibiotics, Jan. 1975, p.87-90 and June 1986, p864-868; in Eur.J.Med.Chem.-Chim.Ther.,1982,17, No. 5, p.482 - 484, and 1980, 15, No. 5, p.475-478, and 1977, 12, No. 1, p.59-62; in J.Chem.Soc., Chem. Commun., 1984, p.323-324; in Chem.Ind. (Milan), 1977, 59(5), p. 385; in Gazetta Chimica Italiana, 106, 1976, p.1107-1110; in Tetrahedron Letters, No. 38, 1974, p. 3431-3432; and in U.S. Patent Nos. 4558040 and 4550121.

The invention will now be further illustrated by the following examples.

Example 1

Preparation of [2-(N-morpholino)sulphonyl]phenyl-ONN -azoxycyanide

Morpholine (9 g; 0.1 mole) in 2 Molar sodium hydroxide solution (80 ml) was treated with 2-nitrobenzenesulphonyl chloride (24.4 g; 0.1 mole). The reaction mixture was stirred for about 2 hours at ambient temperature, acidified and filtered. The product, [2-(N-morpholino)sulphonyl]nitrobenzene, was

washed with hot ethanol. Yield was 8.6 g. mp 176-180 °C.

| Analysis: | Calc. % | C 44.1 | H 4.4 | N 10.3 |
|---|---|---|---|---|
| | Found % | C 43.9 | H 4.4 | N 10.3 |

The product of the preceding step (5.5 g; 0.02 mole) and rhodium on carbon catalyst (0.2 g) were mixed in tetrahydrofuran (100 ml). Hydrazine hydrate (1.2 ml) was added at 0 °C. The reaction mixture was stirred for about 2 hours at ambient temperature, then filtered through celite. The solvent was removed by evaporation. Yield was quantitive. The crude hydroxylamine product (0.02 mole) was dissolved in methanol (30 ml), and the solution was added to a solution of ferric chloride (11g; 0.04 mole) in methanol (30 ml)-/water (80 ml), at 0 °C. The resultant solid compound, [2-(N-morpholino)sulphonyl]nitrosobenzene (4.8 g; c.90%) was dissolved in chloroform (50 ml). Cyanamide (1.3 g) was added, and the mixture treated, at 0 °C, with iodobenzenediacetate (7.2 g) in chloroform (50 ml). The resultant solid, the title compound, was purified by flash chromatography, on silica, using dichloromethane as eluant. Yield was 4.0 g (70%). mp 172-174 °C.

| Analysis: | Calc. % | C 44.6 | H 4.1 | N 18.9 |
|---|---|---|---|---|
| | Found % | C 44.7 | H 4.1 | N 18.3 |

Further compounds were prepared according to procedures similar to those described in the description and in Example 1. Data on these compounds are set out in Table 1 below. In Table 1, reference is made to a compound of the following general formula:

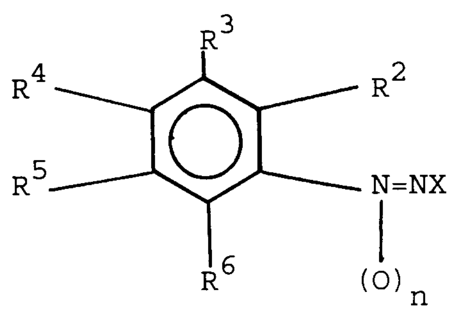

**TABLE 1**

| Ex.No. | $R^2$ | $R^3$ | $R^4$ | $R^5$ | $R^6$ | X | n | Analysis CHN<br>Calc %<br>Found % | mp/bp (°C) | m/e |
|---|---|---|---|---|---|---|---|---|---|---|
| 2 | $SCH_3$ | H | H | Cl | H | CN | 1 | 42.2 2.6 18.5<br>41.9 3.1 18.6 | 158-160 | 227/229 |
| 3 | $SCH_3$ | H | H | H | H | CN | 1 | 49.7 3.6 21.8<br>49.8 3.7 21.7 | 104-106 | 193 |
| 4 | $SCH_2Ph$ | H | H | H | H | CN | 1 | 62.4 4.1 15.6<br>62.5 4.1 15.7 | 118 | 269 |
| 5 | $S(O)CH_3$ | H | H | H | H | CN | 1 | 46.0 3.4 20.1<br>45.1 3.4 19.5 | 140-143 | 209 |
| 6 | $SO_2CH_3$ | H | H | H | H | CN | 1 | 42.7 3.1 18.7<br>43.0 3.1 18.5 | 120-122 | 225 |
| 7 | $SO_2NH_2$ | H | H | H | H | CN | 1 | 37.2 2.7 24.8<br>37.3 3.0 23.6 | 161-165 (decomp.) | 226 |

**TABLE 1** (continued)

| Ex.No. | $R^2$ | $R^3$ | $R^4$ | $R^5$ | $R^6$ | X | n | Analysis CHN Calc % Found % | | | mp/bp (°C) | m/e |
|---|---|---|---|---|---|---|---|---|---|---|---|---|
| 8 | S | H | H | Cl | H | CN | 1 | 47.4<br>47.0 | 3.2<br>3.0 | 16.6<br>16.7 | 108-111 | 253/255 |
| 9 | H | H | $SO_2NH_2$ | H | H | CN | 1 | 37.2<br>37.0 | 2.7<br>2.7 | 24.8<br>24.6 | 178-182 | 226 |
| 10 | $S(O)CH_2Ph$ | H | H | H | H | CN | 1 | 59.0<br>58.8 | 3.9<br>4.0 | 14.8<br>14.9 | 135-137 | 285 |
| 11 | $SO_2CH_2Ph$ | H | H | H | H | CN | 1 | 55.8<br>55.7 | 3.7<br>3.6 | 13.7<br>14.0 | 116-118 | 301 |
| 12 | H | H | $SCH_3$ | H | H | CN | 1 | 49.7<br>49.5 | 3.6<br>3.7 | 21.8<br>21.5 | 100-101 | 193 |
| 13 | $SO_2NHCH_2CO_2C_2H_5$ | H | H | H | H | CN | 1 | 42.3<br>41.9 | 3.9<br>3.9 | 18.0<br>16.2 | 83-86 | 312 |

TABLE 1 (continued)

| Ex.No. | $R^2$ | $R^3$ | $R^4$ | $R^5$ | $R^6$ | X | n | Analysis CHN Calc % Found % | mp/bp (°C) | m/e |
|---|---|---|---|---|---|---|---|---|---|---|
| 14 | $SO_2NH(-4-Cl-Ph)$ | H | H | H | H | CN | 1 | 46.4 2.7 16.7<br>46.3 2.8 15.1 | 48-50 | 336/338 |
| 15 | $SO_2NHCH_2COOH$ | H | H | H | H | CN | 1 | 38.1 2.8 19.7<br>38.6 3.0 18.4 | 145-147 | 286 |
| 16 | H | H | $SO_2NHPh$ | H | H | CN | 1 | 51.6 3.3 18.5<br>51.3 3.5 16.6 | 145-150 | 302 |

<u>Example B1</u>

The fungicidal activity of compounds of the invention was investigated by means of the following tests.

(a) Direct protectant activity against vine downy mildew (Plasmopara viticola; Pvp)

The test is a direct protectant one, using a foliar spray. The lower surfaces of leaves of whole vine plants (cv Cabernet Sauvignon) are sprayed with a solution of active material in 1:1v/v water/acetone containing 0.04%w "Triton X-155" (trade mark) (octylphenol polyoxyethylene surfactant), at a dosage of 1 kilogram of active material per hectare using a track sprayer which delivers 620 litres/ha, and after a subsequent 24 hours under normal glasshouse conditions the lower surfaces of the leaves are inoculated by spraying with an aqueous solution containing $10^4$ zoosporangia/ml. The inoculated plants are kept for 24 hours in a high humidity compartment, 5 days under normal glasshouse conditions and then returned for a further 24 hours to high humidity. Assessment is based on the percentage of leaf area covered by sporulation compared with that on control leaves.

(b) Direct protectant activity against vine grey mould (Botrytis cinerea; Bcp)

The test is a direct protectant one using a foliar spray and is effected as described under (a), with the difference that the leaves are inoculated by spraying with an aqueous solution containing $10^5$ conidia/ml.

(c) Activity against wheat eyespot (Pseudocereosporella herpotrichoides; Ph).

The test is an in vitro one. Samples are prepared wherein 0.7 mls solution containing 2 mg active material dissolved in acetone is evenly dispersed in 20ml molten half-strength potato dextrose agar (formed by dissolving 2g potato extract, 10g dextrose and 7.5g agar in 1 litre of water and sterilising for 15 minutes at 121°C) and the resulting 20ml portions are allowed to set in 9cm petri dishes. The concentration of active material in the resulting samples is 100ppm. Upon setting, two plugs of 5mm diameter taken from the advancing edge of a stock plate of a 3 to 4 week old culture of P.herpotrichoides on full strength potato dextrose agar, incubated at 20-22°C in darkness, are placed, equally spaced on the surface of each sample, mycelial side uppermost. The samples are incubated for 11 days at 20-22°C in darkness before assessment. Diametric growth is measured with the width of the plug subtracted and results compared with growth on a sample wherein 0.7ml acetone containing no active material is dispersed in 20ml half-strength potato agar.

(d) Activity against seedling wheat blight (Fusarium culmorium; Fs)

The test is an anti-sporulant one using a soil drench. Surface sterilised wheat seeds (var Waggoner) are incculated by soaking in an aqueous suspension containing 7 x $10^5$ spores/ml (60mg seed per 80ml suspension) at 22°C for 6 hours. The seeds are then sown in pots (5 per pot) in sand at a depth of 1cm. 1 day after inoculation and planting the active material is applied at a rate of 10kg/ha by pouring on a soil drench (concentration 0.36g/l active material in 12%v/v acetone/water) evenly over the sand. The pots are then transferred to glasshouse, kept at 25°C and watered sparingly. 21 days after inoculation the resulting seedlings are removed from the pots and their roots are gently washed. Visual assessment is made based on lesion development on stem base and upper roots in comparison with control seedlings.

(e) Activity against wheat leafspot (Leptosphaeria nodorum; Ln)

The test is a direct antisporulant one, using a foliar spray. Leaves of wheat plants (cv Mardler), at the single leaf stage, are inoculated by spraying with an aqueous suspension containing 8 x $10^5$ spores/ml. The inoculated plants are kept for 24 hours in a high humidity compartment prior to treatment. The plants are sprayed at a dosage of 1 kg. of active material per hectare using a track sprayer as described under (a). After drying, the plants are kept for 5 days under normal glasshouse conditions, followed by assessment. Assessment is based on the percentage of leaf area covered by sporulation compared with that on leaves of control plants.

(f) Activity against barley powdery mildew (Erysiphe graminis f.sp. hordei; Eg)

The test is a direct antisporulant one, using a foliar spray. Leaves of barley seedlings, cultivar Golden Promise, are inoculated by dusting with mildew conidia one day prior to treatment with the test compound. The inoculated plants are kept overnight at glasshouse ambient temperature and humidity prior to treatment. The plants are sprayed at a dosage of 1kg. of active material per hectare using a track sprayer

as described under (a). After drying, plants are returned to a compartment at ambient temperature and humidity for up to 7 days, followed by assessment. Assessment is based on the percentage of leaf area covered by sporulation compared with that on leaves of control plants.

(g) Activity against tomato early blight (Alternaria Solani;As)

The test is a direct protectant one using a foliar spray. The upper surfaces of leaves of young tomato plants are sprayed with a solution of active material as described in (a) above. After 24 hours under normal glasshouse conditions, the upper surfaces of the leaves are inoculated by spraying with an aqueous suspension containing $10^4$ spores/ml. The inoculated plants are kept for 72 hours in a high humidity compartment and are then removed to lower humidity (50-70% relative humidity). Assessment is made 8 days after inoculation.

(h) Activity against wheat brown rust Puccinia Recondita; Pr)

The test is a direct protectant one using a foliar spray. Wheat seedlings (cv Brigand) are grown to the 1-1.5 leaf stage. The plants are then sprayed with the test compound at a dosage of 1 kg/ha using a track sprayer as described under (a). Test compounds are applied as solutions or suspensions in a mixture of acetone and water (50:50 $^v/_v$) containing 0.04% surfactant ("TWEEN 20" - Trade Mark).

18-24 hours after treatment, the seedlings are inoculated by spraying the plants from all sides with an aqueous spore suspension containing about $10^5$ spores/ml. For 18 hours after inoculation, the plants are kept in high humidity conditions at a temperature of 20-22°C. Thereafter, the plants are kept in ambient glasshouse conditions, that is, in moderate relative humidity and at a temperature of 20°C.

The disease is assessed 10 days after inoculation on the basis of the percentage of the plant covered by sporulating pustules compared with that on the control plants.

(i) Antisporulant activity against vine downy mildew (Plasmopara viticola; Pva)

The test is a direct antisporulant one using a foliar spray. The lower surfaces of leaves of whole vine plants (cv Cabernet Sauvignon) are inoculated by spraying with an aqueous suspension containing $10^4$ zoosporangia/ml 2 days prior to treatment with the test compound. The inoculated plants are kept for 24 hours in a high humidity compartment, and then 24 hours at glasshouse ambiant temperature and humidity. When the plants are dry, infected leaves are sprayed on their lower surfaces with a solution of active material in 1:1 water/acetone containing 0.04% w/w "Triton X-155" (trade mark) (an octylphenol polyethoxylate surfactant). The spraying is carried out with a moving track sprayer with delivers 620 litre/ha, and the concentration of active material is calculated to give an application rate of 1kg/ha. After spraying, the plants are returned to normal glasshouse conditions for 96 hours and are then transferred to the high humidity compartment for 24 hours to induce sporulation, prior to assessment. Assessment is visual and is based on the percentage of the leaf area covered by sporulation compared with that on control leaves.

(j) Activity against rice leaf blast (Pyricularia oryzae;Po)

The test is a direct eradicant one using a foliar spray. The leaves of rice seedlings (about 30 seedlings per pot) are sprayed with an aqueous suspension containing $10^5$ spores/ml 20-24 hours prior to treatment with the test compcund. The incculated plants are kept overnight in high humidity and then allowed to dry before spraying at a dosage of 1kg of active material per hectare using a track sprayer as described under (a). After treatment the plants are kept in a rice compartment at 25-30°C and high humidity. Assessments are made 4-5 days after treatment and are based on the density of necrotic lesions and the degree of withering when compared with control plants.

The results of the above tests are given in Table 2 below

TABLE 2

| Ex.No. | Pvp | Bcp | Ph | Fs | Ln | Eg | As | Pr | Po | Pva |
|---|---|---|---|---|---|---|---|---|---|---|
| 1 | 2 | | 1 | 1 | | | | 1 | | |
| 2 | 2 | 2 | | | | | 2 | | | |
| 3 | | 1 | 1 | 2 | | | 1 | 2 | | 1 |
| 4 | 2 | | | | | | 2 | | | |
| 5 | | | 2 | 2 | | | | | | 1 |
| 6 | 2 | | 2 | 2 | | | 2 | | | |
| 7 | 2 | | 1 | 1 | | | | 1 | | |
| 8 | 2 | | | 1 | | | 2 | 1 | | |
| 9 | 2 | | 1 | 2 | 1 | | 1 | 1 | | |
| 10 | 2 | 2 | 1 | 2 | | | 1 | 1 | | |
| 11 | 2 | | 1 | 2 | | | 2 | 1 | | |
| 12 | | | 1 | 2 | 1 | 2 | | 2 | 2 | |
| 13 | 2 | | 1 | 1 | 1 | 1 | | 1 | | |
| 14 | 2 | | 2 | 2 | | | | | | |
| 15 | 1 | | | | | | | 2 | | |
| 16 | 2 | | 1 | 2 | | | 1 | 1 | | |

The fungicidal activity of certain of the compounds was investigated further, in secondary screening. These revealed that certain compounds have particularly high activity against vine downy mildew. The compounds of Examples 2, 6, 7, 8, 9 and 16 were found to have high activity against Plasmopara viticola, as determined by a translaminar protectant test, in which the upper surfaces of the leaves of whole vine plants are sprayed with a solution of the compound, and the lower leaves are then inoculated with the appropriate Zoosporangia.

Example B2

The activity of compounds of formula I in combating microorganisms was further demonstrated by tests against the following microorganisms.

| | |
|---|---|
| Gram positive bacteria | : Staphylococcus aureus (Sa) (NCIB 6571) |
| | Bacillus subtilis (Bs) |
| Gram negative bacteria | : Escherichia coli (Ec) (NCIB 9517) |
| | Desulfovibrio sp. (Dsp) mixed culture of sulphate-reducing bacteria ex Brent North Sea. |
| Yeast | : Saccharomyces cerevisiae (Sc) (ATCC 9763) |
| Filamentous fungi | : Aspergillus niger (An) (CMI 17454) |
| | Cladosporium resinae (Cr) |
| | Chaetomium globosum (Chg) |

(NCIB = National Collection of Industrial Bacteria, Torry Research Station, P.O. Box 31, 135 Abbey Road, Aberdeen, Scotland. AB9 8DG).

(ATCC = American Type Culture Collection, 12301 Parklawn Drive, Rockville, Maryland 20852, USA). (CMI = Commonwealth Mycological Institute, Ferry Lane, Kew, Surrey, England).

Inocula of the above microorganisms were prepared as follows.

In respect of S. aureus, B. subtilis and E. coli, the microorganisms were cultured in 50ml aliquots of a tryptone soya broth in 250ml conical flasks at 30°C on a rotary shaker at 200rpm for 24 hours. Tryptone soya broth was prepared by adding 17g pancreatic digest of casein, 3g papaic digest of soyabean meal, 5g of sodium chloride, 2.5g of dipotassium hydrogen phosphate and 2.5g of dextrose to 1 litre of distilled water, mixing and sterilising by autoclaving at 121°C for 15 minutes. 1ml aliquots of the resulting cultures were mixed with 99ml of fresh tryptone soya broth and used an inocula for tests.

The sulphate reducing bacteria Desulfovibrio sp. were cultured in a modified Postgate's Medium B for 48 hours at 30°C and used directly as an inoculum. The modified Postgate's Medium B consisted of 0.5g

dipotassium hydrogen phosphate, 1g ammonium chloride, 1g sodium sulphate, 5g sodium lactate, 1g yeast extract, 0.1ml thioglycolic acid, 0.1ml ascorbic acid, 0.5g ferrous sulphate heptahydrate, 750ml aged seawater and 250ml distilled water, pH adjusted to 7.8, divided into aliquots and sterilised by autoclaving at 121°C for 15 minutes.

Inocula of S. cerevisiae were prepared as for S. aureus, B. subtilis and E. coli, but using yeast malt broth in place of the typtone soya broth. The yeast malt broth was prepared by suspending 3g yeast extract, 3g malt extract, 5g peptone and 10g dextrose in 1 litre of distilled water, warming to achieve solution and sterilising by autoclaving at 121°C for 15 minutes.

Inocula of the fungi Aspergillus niger, Cladosporium resinae and Chaetomium globosum were prepared containing $5x10^5$ conidia/ml in a potato dextrose broth. The potato dextrose broth was prepared by suspending 4g potato extract and 20g dextrose in 1 litre of distilled water, warming to achieve solution, separating into aliquots and sterilising by autoclaving at 121°C for 15 minutes.

Stock solutions of the various test compounds were prepared containing 10,000ppm (1%w) compound in distilled water (in cases where the compound did not dissolve, up to 4%v/v acetone was included in the stock solution). Tests showed that such levels of acetone had no observable adverse effects on growth of the above listed microorganisms). Test procedures were as follows.

(i) Sulphate-reducing bacteria test dilution series

Duplicate aliquots (9.9ml) of dilution series from the stock solutions of each compound were prepared in a modified Postgate's Medium B described above. These contained 100ppm compound. These were then inoculated with 0.1ml of inoculum. After incubation at 30°C, the presence or absence of growth - as indicated by a blackening of the medium due to ferrous sulphide production - was recorded at 2, 5 and 10 days. Compounds active at 100ppm were further tested at the lower concentrations of 5, 10, 25 and 50ppm.

(ii) Dilution series for other test bacteria, yeast and filamentous fungi

Dilution series from the stock solution of each compound were prepared in sterile distilled water. These contained 50, 100, 500 and 1000ppm compound. To duplicate wells of a compartmentalised square petri dish 0.3ml of each test compound concentration was added together with 2.7ml of one of the microbial inocula described above. This gave final concentrations of the test compound of 5, 10, 50 and 100ppm.

After incubation, in the dark, at 30°C the wells were examined for the presence or absence of growth. Bacterial and yeast cultures were examined after 24 hours and 72 hours incubation and the fungi cultures after 3, 7 and 10 days.

By means of the above tests minimum inhibitory concentrations were determined for the various test compounds. These are given in Table 3 below.

TABLE 3

| Minimum inhibitory concentration (ppm) | | | | | | | | |
|---|---|---|---|---|---|---|---|---|
| Ex.No. | Sa | Bs | Ec | Dsp | Sc | An | Cr | Chg |
| 1 | >100 | 100 | >100 | >100 | 100 | >100 | >100 | 100 |
| 3 | >100 | >100 | >100 | 50 | 50 | 50 | 50 | <100 |
| 5 | >100 | 50 | >100 | 50 | 100 | 100 | 100 | <100* |
| 9 | 50 | >100 | 50-100 | 5-10 | 50 | >100 | 50 | 50 |
| 10 | >100 | 100 | >100 | >100 | 100 | 50 | 100 | 50 |
| 13 | 100 | 100 | 100 | 100 | >100 | >100 | >100 | >100 |
| 16 | >100 | >100 | >100 | 100 | 25 | >100 | >100 | >100 |

* only tested at 100 ppm.

## Example B3

Compounds of the invention were found to have nematicidal activity, in particular the compound of Example 12. It was tested against the rice root nematode Meloidogyne graminicola in a water screen whereby the effect of retaining the nematodes in a dilute aqueous solution of the compcund was studied, and in a soil drench test in which the propensity of these nematodes to cause knots in the roots of grain sorghum S.bicolor was studied as a function of concentration of the compound applied to the soil. The compound was found to have activity against the nematodes in each of the tests. In the water screen test it

was determined that the minimum inhibitory concentration of the compound was 0.72ppm, and in the soil drench test, 10ppm.

## Claims

1. A method of combating a bacterium and/or nematode at a locus, which method comprises treating a locus with a compound of the general formula

   R-N=NX (I)

   or an N-oxide thereof, wherein R represents a substituted phenyl group, at least one substituent thereof being a group of general formula $-S(O)_m-Z$ where m represents 0, 1 or 2 and Z represents a hydrogen atom or an optionally substituted alkyl, cycloalkyl, aryl, aralkyl or amino group; and X represents a cyano group, a group -COOH or a salt, ester or amido derivative thereof.

2. A method as claimed in Claim 1, wherein, in the compound of formula I, X represents a cyano group.

3. A method as claimed in Claim 1 or Claim 2, wherein, in the compound of formula I, R represents a phenyl group, wherein Z of the substituent $-S(O)_m-Z$represents an alkyl group, a cycloalkyl group, a benzyl group, or a group of general formula $NR^1R^2$ where each of $R^1$ and $R^2$ independently represents a hydrogen atom, or an optionally substituted phenyl or alkyl group, or together may represent an alkylene chain which is optionally interrupted by an oxygen atom.

4. A method as claimed in Claim 3, wherein, in the compound of formula I, Z represents a $C_{1-4}$ alkyl group, a $C_{3-6}$ cycloalkyl group, a benzyl group or a group of general formula $-NR^1R^2$ wherein $R^1$ represents a hydrogen atom and $R^2$ represents a hydrogen atom, or a phenyl or halophenyl group, or an alkyl group optionally substituted by a group of general formula $-CO_2R^3$ where $R^3$ represents a hydrogen atom or an alkyl group; or $R^1$ and $R^2$ together represent a group of formula $-(CH_2)_2-O-(CH_2)_2-$.

5. A method as claimed in any one of the preceding Claims, wherein, in the compound of formula I, R represents a group of general formula

   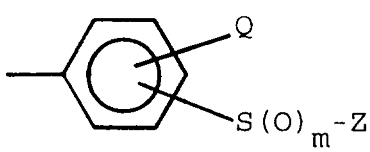

   wherein Q represents a halogen or hydrogen atom.

6. A compound of the general formula

   R-N=NX (I)

   or an N-oxide thereof, where R represents a substituted phenyl group, at least one substituent thereof being a group of general formula $-S(O)_m-Z$ where m represents 0, 1 or 2 and Z represents a hydrogen atom or an optionally substituted alkyl, cycloalkyl, aryl, aralkyl or amino group; and X represents a cyano group, a group -COOH or a salt, ester or amido derivative thereof, provided that in an un-N-oxidised compound of formula I when m is 2, Z is other than a methyl group.

7. A process for the preparation of a compound of the general formula I, as defined in Claim 1, which process comprises reacting a compound of the general formula

   R-N=O (II)

   with cyanamide, to form an N-oxide compound of formula I wherein X represents a cyano group; and

optionally derivatising that compound to produce a further compound of formula I.

**8.** A process for the preparation of a compound of general formula I, as claimed in Claim 6, which comprises reacting a compound of the general formula

$R\text{-}N_2^+X^-$ (IV)

where $X^-$ is an anion derived from a mineral acid, with an alkali metal cyanide, to form an un-N-oxidised compound of general formula I; and optionally derivatising that compound to provide a further compound of general formula I.

**9.** A biocidal composition which comprises a carrier and, as active ingredient, a compound as claimed in Claim 6.

**10.** A method of combating a fungus, and/or yeast at a locus, which method comprises treating a locus with a compound as claimed in Claim 6 or with a composition as claimed in Claim 9.

**11.** Use as a bactericide and/or a nematicide of a compound of general formula I as defined in Claim 1.

**12.** Use as a biocide of a compound as claimed in Claim 6 or of a composition as claimed in Claim 9.

**Patentansprüche**

**1.** Ein Verfahren zum Bekämpfen eines Bakteriums und/oder einer Nematode an einem Ort, wobei das Verfahren umfaßt das Behandeln eines Ortes mit einer Verbindung der allgemeinen Formel

R - N = NX (I)

oder deren N-Oxid, in der R eine substituierte Phenylgruppe darstellt, wobei mindestens ein Substituent eine Gruppe der allgemeinen Formel $-S(O)_m-Z$ ist, wobei m 0, 1 oder 2 darstellt und Z ein Wasserstoffatom oder eine gegebenenfalls substituierte Alkyl-, Cycloalkyl-, Aryl-, Aralkyl- oder Aminogruppe darstellt; und X eine Cyanogruppe, eine Gruppe -COOH oder ein Salz, Ester oder Amidoderivat davon darstellt.

**2.** Ein Verfahren, wie in Anspruch 1 beansprucht, wobei bei der Verbindung der Formel I, X eine Cyanogruppe darstellt.

**3.** Ein Verfahren, wie in Anspruch 1 oder 2 beansprucht, wobei bei der Verbindung der Formel I R eine Phenylgruppe darstellt, wobei Z des Substituenten von $-S(O)_m-Z$ eine Alkylgruppe, eine Cycloalkylgruppe, eine Benzylgruppe oder eine Gruppe der allgemeinen Formel $NR^1R^2$ darstellt, wobei jedes $R^1$ und $R^2$ unabhängig voneinander ein Wasserstoffatom oder eine gegebenenfalls substituierte Phenyl- oder Alkylgruppe darstellen oder zusammen eine Alkylenkette darstellen können, die gegebenenfalls durch ein Sauerstoffatom unterbrochen ist.

**4.** Ein Verfahren, wie in Anspruch 3 beansprucht, wobei bei der Verbindung der Formel I Z eine $C_{1-4}$-Alkylgruppe, eine $C_{3-6}$-Cycloalkylgruppe, eine Benzylgruppe oder eine Gruppe der allgemeinen Formel $-NR^1R^2$ darstellt, wobei $R^1$ ein Wasserstoffatom darstellt und $R^2$ ein Wasserstoffatom oder eine Phenyl- oder Halogenphenylgruppe oder eine Alkylgruppe darstellt, die gegebenenfalls durch eine Gruppe der allgemeinen Formel $-CO_2R^3$ substituiert ist, wobei $R^3$ ein Wasserstoffatom oder eine Alkylgruppe darstellt; oder $R^1$ und $R^2$ zusammen eine Gruppe der Formel $-(CH_2)_2-O-(CH_2)_2-$ darstellen.

**5.** Ein Verfahren, wie in irgendeinem der vorstehenden Ansprüche beansprucht, wobei bei der Verbindung der Formel I R eine Gruppe der allgemeinen Formel

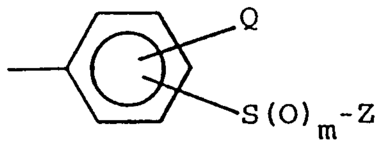

darstellt, in der Q ein Halogen- oder Wasserstoffatom darstellt.

**6.** Eine Verbindung der allgemeinen Formel

R - N = NX (I)

oder deren N-Oxid, in der R eine substituierte Phenylgruppe darstellt, wobei mindestens ein Substituent davon eine Gruppe der allgemeinen Formel $-S(O)_m-Z$ ist, wobei m 0, 1, oder 2 darstellt und Z ein Wasserstoffatom oder eine gegebenenfalls substituierte Alkyl-, Cycloalkyl-, Aryl-, Aralkyl- oder Aminogruppe darstellt; und X eine Cyanogruppe, eine Gruppe -COOH oder ein Salz, Ester oder Amidoderivat davon darstellt, mit der Maßgabe, daß bei einer nicht als N-Oxid vorliegenden Verbindung der Formel I, wenn m 2 ist, Z ein anderer Rest als eine Methylgruppe ist.

**7.** Ein Verfahren zur Herstellung einer Verbindung der allgemeinen Formel I, wie in Anspruch 1 definiert, wobei das Verfahren umfaßt das Umsetzen einer Verbindung der allgemeinen Formel

R - N = O (II)

mit Cyanamid zur Bildung einer N-Oxid-Verbindung der Formel I, wobei X eine Cyanogruppe darstellt; und gegebenenfalls Derivatisieren jener Verbindung zur Erzeugung einer weiteren Verbindung der Formel I.

**8.** Ein Verfahren zur Herstellung einer Verbindung der allgemeinen Formel I, wie in Anspruch 6 beansprucht, das umfaßt ein Umsetzen einer Verbindung der allgemeinen Formel

$R - N_2^+X^-$ (IV)

wobei $X^-$ ein sich von einer Mineralsäure ableitendes Anion ist, mit einem Alkalimetallcyanid zur Bildung einer nicht das N-Oxid umfassenden Verbindung der allgemeinen Formel I; und gegebenenfalls Derivatisieren jener Verbindung zur Verfügungstellung einer weiteren Verbindung der allgemeinen Formel I.

**9.** Eine biozide Zusammensetzung, die umfaßt ein Trägermaterial und - als aktiven Bestandteil - eine Verbindung, wie in Anspruch 6 beansprucht.

**10.** Ein Verfahren zur Bekämpfung eines Fungus und/oder einer Hefe an einem Ort, wobei das Verfahren ein Behandeln eines Ortes mit einer Verbindung, wie in Anspruch 6 beansprucht, oder mit einer Zusammensetzung, wie in Anspruch 9 beansprucht, umfaßt.

**11.** Verwendung einer Verbindung der allgemeinen Formel I, wie in Anspruch 1 definiert, als ein Bakterizid und/oder ein xNematizid.

**12.** Verwendung einer Verbindung, wie in Anspruch 6 beansprucht, oder einer Zusammensetzung, wie in Anspruch 9 beansprucht, als ein Biozid.

**Revendications**

**1.** Procédé pour lutter contre une bactérie et/ou un nématode sur un locus, ledit procédé comprenant le traitement d'un locus avec un composé de formule générale

R-N = NX (I)

ou un N-oxyde de celui-ci, dans laquelle R représente un groupe phényle substitué, au moins un substituant de celui-ci étant un groupe de formule générale $-S(O)_m-Z$, où m représente 0, 1 ou 2 et Z représente un atome d'hydrogène ou un groupe alkyle, cycloalkyle, aryle, aralkyle ou amino, substitué en option; et X représente un groupe cyano, un groupe -COOH ou un dérivé salin, ester ou amido de ces groupes.

2. Procédé selon la revendication 1, dans lequel, dans le composé de formule I, X représente un groupe cyano.

3. Procédé selon l'une des revendications 1 ou 2, dans lequel, dans le composé de formule I, R représente un groupe phényle, dans lequel Z du substituant $-S(O)_m-Z$représente un groupe alkyle, un groupe cycloalkyle, un groupe benzyle ou un groupe de formule générale $NR^1R^2$, où $R^1$ et $R^2$ représentent chacun, indépendamment l'un de l'autre, un atome d'hydrogène, ou un groupe phényle ou alkyle éventuellement substitué, ou conjointement peuvent représenter une chaîne alkylène pouvant être interrompue par un atome d'oxygène.

4. Procédé selon la revendication 3, dans lequel, dans le composé de formule I, Z représente un groupe alkyle en $C_{1-4}$, un groupe cycloalkyle en $C_{3-6}$, un groupe benzyle ou un groupe de formule générale $-NR^1R^2$, où $R^1$ représente un atome d'hydrogène et $R^2$ représente un atome d'hydrogène, ou un groupe phényle ou halogénophényle, ou un groupe alkyle éventuellement substitué par un groupe de formule générale $-CO_2R^3$, où $R^3$ représente un atome d'hydrogène ou un groupe alkyle; ou $R^1$ et $R^2$ représentent ensemble un groupe de formule $-(CH_2)_2-O-(CH_2)_2-$.

5. Procédé selon l'une quelconque des revendications précédentes, dans lequel, dans le composé de formule I, R représente un groupe de formule générale

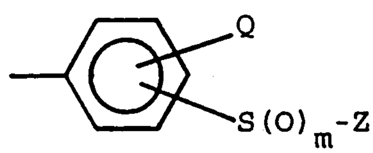

où Q représente un atome d'halogène ou d'hydrogène.

6. Composé de formule générale

R-N = NX (I)

ou un N-oxyde de celui-ci, dans laquelle R représente un groupe phényle substitué, au moins un substituant de celui-ci étant un groupe de formule générale $-S(O)_m-Z$, où m représente 0, 1 ou 2 et Z représente un atome d'hydrogène ou un groupe alkyle, cycloalkyle, aryle, aralkyle ou amino, substitué en option; et X représente un groupe cyano, un groupe -COOH ou un dérivé salin, ester ou amido de ces groupes, à condition que dans un composé non-N-oxydé de formule I, lorsque m est 2, Z soit autre qu'un groupe méthyle.

7. Procédé pour la préparation d'un composé de formule générale I, tel que défini dans la revendication 1, lequel procédé comprend la réaction d'un composé de formule générale

R-N = O (II)

avec un cyanamide, pour former un composé N-oxydé de formule I, où X représente un groupe cyano; et en option la formation d'un dérivé de ce composé pour produire un autre composé de formule I.

8. Procédé pour la préparation d'un composé de formule générale I selon la revendication 6, comprenant la réaction d'un composé de formule générale

$R-N_2^+X^-$ (IV)

où $X^-$ est un anion dérivé d'un acide minéral, avec un cyanure de métal alcalin, pour former un composé de formule générale I non-N-oxydé; et en option la formation d'un dérivé de ce composé pour fournir un autre composé de formule générale I.

**9.** Composition biocide comprenant un véhicule ou support et, en tant que constituant actif, un composé selon la revendication 6.

**10.** Procédé pour lutter contre un mycète et/ou une levure sur un locus, lequel procédé comprend le traitement d'un locus avec un composé selon la revendication 6 ou avec une composition selon la revendication 9.

**11.** Utilisation en tant que bactéricide et/ou nématicide d'un composé de formule générale I tel que défini dans la revendication 1.

**12.** Utilisation en tant que biocide d'un composé selon la revendication 6 ou d'une composition selon la revendication 9.